(19) Europäisches Patentamt
European Patent Office
Office européen des brevets

(11) Veröffentlichungsnummer: **0 292 985 B1**

(12) # EUROPÄISCHE PATENTSCHRIFT

(45) Veröffentlichungstag der Patentschrift: **25.03.92**

(51) Int. Cl.[5]: **C07C 205/31**, C07C 201/12

(21) Anmeldenummer: **88108483.4**

(22) Anmeldetag: **27.05.88**

(54) **Verfahren zur Herstellung von 2,4-Dinitrophenylethern.**

(30) Priorität: **29.05.87 DE 3718175**
**24.06.87 DE 3720836**

(43) Veröffentlichungstag der Anmeldung:
**30.11.88 Patentblatt 88/48**

(45) Bekanntmachung des Hinweises auf die Patenterteilung:
**25.03.92 Patentblatt 92/13**

(84) Benannte Vertragsstaaten:
**BE CH DE FR GB IT LI NL**

(56) Entgegenhaltungen:
**DE-A- 3 335 186**
**DE-A- 3 519 982**
**DE-A- 3 519 983**
**DE-C- 379 881**
**DE-C- 386 618**
**DE-C- 479 831**

(73) Patentinhaber: **HOECHST AKTIENGESELLSCHAFT**
**Postfach 80 03 20**
**W-6230 Frankfurt am Main 80(DE)**

(72) Erfinder: **Heck, Dieter**
**An der Nassburg 16**
**W-6369 Nidderau(DE)**
Erfinder: **Heise, Hartmut, Dr.**
**Am Rehsteig 8**
**W-6232 Bad Soden am Taunus(DE)**
Erfinder: **Hintzmann, Manfred, Dr.**
**Sachsenring 48**
**W-6238 Hofheim am Taunus(DE)**

EP 0 292 985 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung ist ein Verfahren zur Herstellung von 2,4-Dinitrophenylethern durch wasserfreie Umsetzung von 2,4-Dinitrochlorbenzol mit einem Alkohol in Gegenwart von wasserfreien Alkalimetallcarbonaten.

Die Herstellung von 2,4-Dinitrophenylethern aus 2,4-Dinitrochlorbenzol ist im Prinzip literaturbekannt. Diskutiert werden dabei hauptsächlich folgende Herstellungsverfahren:

Ausgehend von 2,4-Dinitrochlorbenzol wird die Umsetzung zum 2,4-Dinitrophenylether in dem für die Etherbildung benötigten Alkohol durchgeführt unter Zugabe von festem Natriumhydroxid oder Kaliumhydroxid oder durch Zugabe von konzentrierten wäßrigen Lösungen dieser Hydroxide (Ber. 12, 765; R 21 439; Bl 27, 105; EP 0 011 048).

Eine weitere Methode zur Herstellung von 2,4-Dinitrophenylethern besteht in der Umsetzung von 2,4-Dinitrochlorbenzol mit einem Alkalimetallalkoholat in dem entsprechenden Alkohol oder in der Umsetzung mit Alkalimetallen in dem für die Etherbildung benötigten Alkohol (Ber. 8, 666).

Alle bisher beschriebenen Herstellungsverfahren haben den Nachteil, daß sie entweder nicht allgemein anwendbar, technisch aufwendig oder sicherheitstechnisch unbefriedigend sind oder aber durch die Bildung von Nebenprodukten, insbesondere von 2,4-Dinitrophenol, unzureichende Ausbeuten und Qualitäten erbringen. So ist die Umsetzung von 2,4-Dinitrochlorbenzol mit einem Alkalimetallalkoholat in dem dem Alkoholat entsprechenden Alkohol zu dem entsprechenden 2,4-Dinitrophenylether praktisch auf die niederen Alkanole beschränkt, weil sich nur von diesen ausreichend konzentrierte Alkoholat-Lösungen herstellen lassen.

Auch die Verwendung von Alkalimetallen, wie Natrium oder Kalium, in dem für die Etherbildung benötigten Alkohol kommt wegen der gleichzeitigen Entwicklung von Wasserstoff aus Sicherheitsgründen für eine großtechnische Durchführung des Verfahrens nicht in Frage.

Bei dem in der Praxis am häufigsten angewandten Verfahren zur Herstellung von 2,4-Dinitrophenylethern aus 2,4-Dinitrochlorbenzol werden neben dem zur Etherbildung und als Lösungsmittel benötigten Alkohol festes Alkalimetallhydroxid oder konzentrierte wäßrige Lösungen davon bei Temperaturen zwischen 10° C und 20° C verwendet. Da bei diesem Herstellungsverfahren in Abhängigkeit von dem verwendeten Alkohol stets mehr oder weniger große Mengen an 2,4-Dinitrophenol entstehen, liegen die in der Literatur für 2,4-Dinitrophenylether angegebenen Ausbeuten selten höher als 90 %. Der wesentliche Nachteil bei der technischen Durchführung dieses Verfahrens ist neben der Ausbeuteminderung durch die Bildung von 2,4-Dinitrophenol dessen Entsorgung. Ferner liegt während der Chlor-Austausch-Reaktion 2,4-Dinitrophenol überwiegend als Alkalimetallsalz vor, welches, da es im Lösungsmittel praktisch unlöslich ist, sich meist als Anbackung an der Kesselwandung zu erkennen gibt. Die Alkalimetallsalze von 2,4-Dinitrophenol sind jedoch, besonders in trockenem Zustand, sehr instabil, so daß derartige Reaktionen aus Sicherheitsgründen bei möglichst tiefer Temperatur durchgeführt werden müssen.

Es wurde überraschenderweise gefunden, daß man 2,4-Dinitrophenylether der allgemeinen Formel (1)

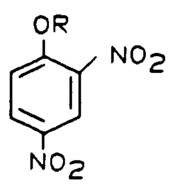

(1),

in welcher R eine Alkyl($C_1$-$C_6$)- oder Alkoxy($C_1$-$C_4$)-alkyl($C_1$-$C_4$)-Gruppe bedeutet, in vorteilhafter Weise und in hohen Ausbeuten herstellen kann, indem man 1 Mol 2,4-Dinitrochlorbenzol in dem für die Etherbildung benötigten wasserfreien Alkohol der allgemeinen Formel (2)

R - OH (2),

in welcher R die vorstehend genannte Bedeutung hat, in Gegenwart von 1,0 bis 3,0 Mol, vorzugsweise 1,05 bis 1,8 Mol, eines wasserfreien Alkalimetallcarbonats, vorzugsweise wasserfreiem Kaliumcarbonat, bei Temperaturen von 20° C bis 150° C, vorzugsweise 40° C bis 120° C, ggf. unter Druck (in Abhängigkeit vom eingesetzten Alkohol der genannten Formel (2)), umsetzt.

Was die einzusetzende Menge an Alkohol der genannten Formel (2) anbelangt, so hängt diese in erheblichem Maße vom eingesetzten Alkohol selbst und der Löslichkeit des angewandten Alkalimetallcarbonats im Alkohol ab. Stöchiometrisch gesehen reicht bereits 1 Mol an Alkohol der Formel (2), bezogen auf

eingesetztes 2,4-Dinitrochlorbenzol, aus. Hierbei kann jedoch eine so schwer rührbare Suspension von Alkalimetallcarbonat im Alkohol der Formel (2), d.h. im Reaktionsgemisch anfallen, daß eine technisch Ausführung des Verfahrens erschwert oder praktisch unmöglich wird.

Man kann zwar zur Vermeidung dieses Nachteils auch so arbeiten, daß man das Molverhältnis 2,4-Dinitrochlorbenzol : Alkohol (Formel (2)) = 1 : 1 oder beispielsweise 1 : 1,10 anwendet und zur Erzielung einer ausreichend rührbaren Suspension noch ein inertes Verdünnungsmittel, wie beispielsweise Toluol oder Xylol, zusetzt. Diese Arbeitsweise hat jedoch den Nachteil, daß man das angewandte inerte Verdünnungsmittel anschließend in gesonderten Verfahrensschritten entfernen muß.

Zur Durchführung des erfindungsgemäßen Verfahrens sei im einzelnen noch folgendes bemerkt: Es hat sich als zweckmäßig erwiesen, 2,4-Dinitrochlorbenzol, gelöst in dem zur Etherbildung benötigten Alkohol, zu einer gerührten und auf 50 °C erwärmten Suspension von Kaliumcarbonat in dem genannten Alkohol derart zulaufen zu lassen, daß eine Reaktionstemperatur von 60 °C nicht überschritten wird. Je nach verwendetem Alkohol wird die Reaktion in der Regel in 4-5 Stunden bei 60 °C bzw. bei Rückflußtemperatur zu Ende geführt. In Abhängigkeit von der Art des eingesetzten Alkohols und von der Art und Menge des eingesetzten Alkalimetallcarbonats sowie von der Reaktionstemperatur kann die Reaktionszeit auch kürzer oder länger sein.

Man kann aber auch so verfahren, daß man einer gerührten und auf 50 °C erwärmten Lösung von 2,4-Dinitrochlorbenzol in dem zur Etherbildung benötigten Alkohol mindestens 1 Mol Kaliumcarbonat pro Mol 2,4-Dinitrochlorbenzol in Anteilen hinzufügt und bis zum vollständigen Umsatz nachrührt.

Bei Alkoholen, die mit 2,4-Dinitrochlorbenzol in Gegenwart von Kaliumcarbonat nur schwach exotherm reagieren, ist es vorteilhafter, sämtliche Reaktionspartner auf einmal zusammenzugeben und bis zum vollständigen Umsatz unter Rückfluß zu erhitzen.

Besonders überraschend beim erfindungsgemäßen Verfahren ist, daß bei Verwendung von mindestens 1 Mol Kaliumcarbonat pro Mol 2,4-Dinitrochlorbenzol praktisch kein 2,4-Dinitrophenol gebildet wird.

Führt man dagegen die gleiche Reaktion mit festem Natrium-oder Kaliumhydroxid durch, so kann der Chlor-Austausch nur unter Bildung von 1 Mol Wasser pro Mol 2,4-Dinitrochlorbenzol erfolgen gemäß dem Reaktionsschema

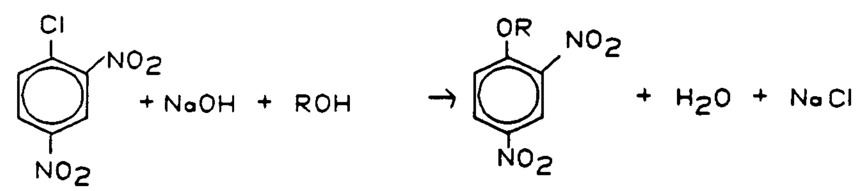

Anwesenheit von Wasser im Reaktionsgemisch bedeutet, daß ein Teil des Chlors im 2,4-Dinitrochlorbenzol gegen die OH-Gruppe ausgetauscht wird, was zur Bildung des unerwünschten 2,4-Dinitrophenols und damit zu Ausbeuteverlusten an Zielprodukt führt.

Ein weiterer überraschender Aspekt des Verfahrens liegt in der leichten Beherrschbarkeit der Reaktion. Während der Chloraustausch in Gegenwart von Alkalimetallhydroxiden oder Alkalimetallalkoholaten stark exotherm ist, so daß besondere Vorkehrungen getroffen werden müssen, damit die Reaktion sicher und temperaturkontrolliert durchgeführt werden kann, ist der Chloraustausch in Gegenwart von Kaliumcarbonat erheblich weniger exotherm. Selbst beim Zusammengeben aller Reaktionspartner auf einmal steigt die Temperatur - ohne äußere Kühlung - von Raumtemperatur nur langsam auf maximal 70 °C.

Die nach dem erfindungsgemäßen Verfahren hergestellten 2,4-Dinitrophenylether werden in einer Ausbeute von 95 - 98 % der Theorie und in einer Reinheit von 98 - 99 % isoliert. Die aus dem Reaktionsgemisch entnommenen und gaschromatographisch untersuchten Proben zeigen, daß die Bildung von 2,4-Dinitrophenol bei Temperaturen bis 60 °C weniger als 0,2 %, bezogen auf eingesetztes 2,4-Dinitrochlorbenzol, beträgt.

Die 2,4-Dinitrophenylether der vorstehend genannten allgemeinen Formel (1) stellen wertvolle Vorprodukte zur Herstellung von Dispersionsfarbstoffen dar.

Die nachstehenden Beispiele dienen zur Erläuterung der Erfindung, ohne sie darauf zu beschränken.

Beispiel 1

In ein Gemisch aus 202,5 g (1 Mol) 2,4-Dinitrochlorbenzol und 157 ml Methylglykol werden innerhalb 1 Stunde 172,7 g (1,25 Mol) wasserfreies Kaliumcarbonat in Anteilen derart eingetragen, daß die Temperatur

während dieser Zeit von 25 ° C auf 60 ° C steigt. Anschließend wird 4 Stunden bei 60 ° C gerührt.

Die dem Reaktionsgemisch dann entnommenen und gaschromatographisch untersuchten Proben zeigen, daß vollständiger Umsatz eingetreten ist und daß nur 0,13 % des eingesetzten 2,4-Dinitrochlorbenzols zu 2,4-Dinitrophenol reagiert haben.

Danach wird das Reaktionsgemisch mit 320 ml Wasser verdünnt, wobei die Salze weitgehend in Lösung gehen und 2,4-Dinitro-methoxyethoxybenzol ausfällt. Das auf einer Nutsche isolierte Produkt wird mit Wasser neutral gewaschen. Nach Trocknen im Vakuum werden 234,7 g 2,4-Dinitromethoxyethoxybenzol in einer Reinheit von > 99 % erhalten, was einer Ausbeute von 97,0 % der Theorie entspricht.

Beispiel 2

Zu einer Suspension von 172,7 g (1,25 Mol) wasserfreiem Kaliumcarbonat in 125 ml Methylglykol wird langsam eine 40 - 45 ° C warme Lösung von 202,5 g (1 Mol) 2,4-Dinitrochlorbenzol in 32 ml Methylglykol so zugetropft, daß eine Reaktionstemperatur von 60 ° C nicht überschritten wird.Anschließend läßt man noch 4 Stunden bei 60 ° C nachrühren.

Die Aufarbeitung des angefallenen Reaktionsgemisches erfolgt wie in Beispiel 1 beschrieben.

In der DE-PS 479 831 wird die Herstellung von 2,4-Dinitrophenoxyethanol durch Umsetzung von 2,4-Dinitrochlorbenzol mit Glykol in Gegenwart von Natriumcarbonat als Stand der Technik erwähnt, wobei keine näheren Reaktionsangaben gemacht werden.

Das in der DE-PS 379 881 beschriebene Verfahren zur Herstellung aromatischer Glykolether von aromatischen Nitrooxyverbindungen geht von Mononitromonohalogenbenzolen aus, die in Gegenwart von Kupferkatalysatoren und Alkalicarbonaten mit Ethylenglykol umgesetzt werden. Angaben über Ausbeute und anfallende Nebenprodukte werden nicht gemacht.

Bei dem in der DE-OS 3 335 186 beschriebenen Verfahren zur Herstellung von Dinitrophenylethern handelt es sich um ein Umetherungsverfahren in Anwesenheit katalytischer Mengen Base. Als Basen kommen sowohl anorganische Basen, wie Alkalihydroxide oder -carbonate, als auch organische Basen, wie Pyridin oder Diethylamin, zur Anwendung, und zwar in einer Menge von 0,5 bis 0,1 Mol pro Mol Einsatzverbindung. Einsatzverbindung ist in allen Fällen ein 1-Methoxy-2,6-dintro-4-alkylbenzol, bei dem die Methoxygruppe im Verlauf der Reaktion durch eine andere Alkoxygruppe ersetzt wird. Im Vergleichsbeispiel 1 wird eine dem erfindungsgemäßen Verfahren ähnliche Reaktion beschrieben. Pro Mol Dinitrochlorbenzol erfolgt die Umsetzung mit sek.-Butanol in Gegenwart von nur 0,5 Mol Kaliumcarbonat. Gemäß der Gleichung

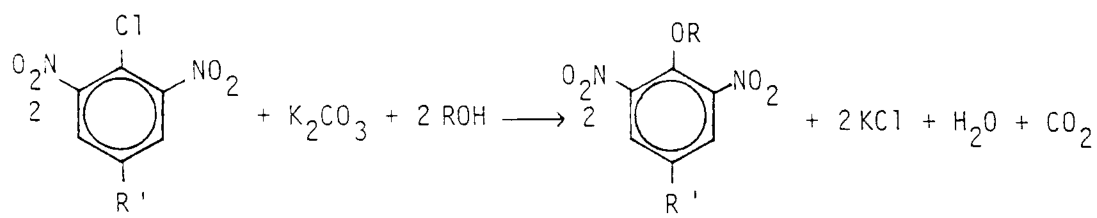

entsteht hierbei bei den angewandten Molverhältnissen zwangsläufig Wasser und damit auch Phenol, was zu Lasten der Ausbeute an Zielprodukt (62 %) geht.

Gewonnen werden 234,2 g 2,4-Dinitro-methoxyethoxybenzol, was einer Ausbeute von 96,7 % der Theorie entspricht. Der Anteil an unerwünschtem 2,4-Dinitrophenol beträgt 0,16 %, bezogen auf eingesetztes 2,4-Dinitrochlorbenzol.

Beispiel 3

Zu einer Suspension von 148 g (1,07 Mol) wasserfreiem Kaliumcarbonat in 125 ml Methylglykol wird langsam eine 40 bis 45 ° C warme Lösung von 202,5 g (1 Mol) 2,4-Dinitrochlorbenzol in 32 ml Methylglykol derart zugetropft, daß eine Reaktionstemperatur von 60 ° C nicht überschritten wird. Anschließend läßt man bei 60 ° C nachrühren. Hierbei zeigt sich, daß selbst nach einer Reaktionsdauer von insgesamt 20 Stunden noch kein vollständiger Umsatz erfolgt ist.

Nach Aufheizen auf 100 ° C und nach zweistündigem Rühren bei dieser Temperatur ist kein 2,4-Dinitrochlorbenzol mehr nachweisbar.

Gewonnen werden 223,6 g 2,4-Dinitro-methoxyethoxybenzol, was einer Ausbeute von 92,3 % der Theorie entspricht.

Der Anteil an unerwünschtem 2,4-Dinitrophenol beträgt 1,3 %, bezogen auf eingesetztes 2,4-Dinitrochlorbenzol.

Beispiel 4

In ein Gemisch aus 160 ml Methanol und 205,5 g (1 Mol) 2,4-Dinitrochlorbenzol werden innerhalb von 30 Minuten 172,7 g (1,25 Mol) Kaliumcarbonat in Anteilen derart eingetragen, daß die Temperatur auf ca. 60 °C ansteigt. Anschließend wird 2 Stunden unter Rückfluß erhitzt. Danach ist vollständiger Umsatz eingetreten.

Das anschließend durch Zugabe von 320 ml Wasser ausgefällte, auf einer Nutsche isolierte und mit Wasser neutral gewaschene Produkt ergibt nach dem Trocknen 195,6 g 2,4-Dinitroanisol vom Schmelzpunkt 94 - 95 °C, was einer Ausbeute von 98,8 % der Theorie entspricht.

Beispiel 5

In ein Gemisch aus 300 ml absolutem Ethanol und 202,5 g (1 Mol) 2,4-Dinitrochlorbenzol werden innerhalb von 30 Minuten 221 g (1,6 Mol) Kaliumcarbonat eingetragen. Anschließend wird 6 Stunden unter Rückfluß erhitzt.

Das analog Beispiel 4 gefällte und isolierte Produkt ergibt nach dem Trocknen 206,8 g 2,4-Dinitrophenetol vom Schmelzpunkt 86 - 87 °C, was einer Ausbeute von 97,5 % der Theorie entspricht.

Beispiel 6

Ein Gemisch aus 320 ml 1-Propanol, 202,5 g (1 Mol) 2,4-Dinitrochlorbenzol und 235 g (1,7 Mol) wasserfreiem Kaliumcarbonat wird 8 Stunden auf Rückflußtemperatur erhitzt. Nach Aufarbeitung des Ansatzes gemäß Beispiel 4 werden 220,4 g 1-Propoxy-2,4-dinitrobenzol vom Schmelzpunkt 34 - 35 °C erhalten, was einer Ausbeute von 97,5 % der Theorie entspricht.

Beispiel 7

Ein Gemisch aus 320 ml 1-Butanol, 202,5 g (1 Mol) 2,4-Dinitrochlorbenzol und 235 g (1,7 Mol) Kaliumcarbonat wird bis zum vollständigen Umsatz des Dinitrochlorbenzols auf Rückflußtemperatur erhitzt. Die Reaktion ist nach 4 Stunden beendet. Nach Zugabe von 350 ml Wasser trennt sich das angefallene Reaktionsgemisch in 3 Phasen, von denen die untere (Zielprodukt) und die obere Phase (1-Butanol) vereinigt werden. Die mittlere wäßrige Phase wird verworfen. Nach Abdestillieren des Butanols werden 231,9 g 1-Butoxy-2,4-dinitrobenzol in Form eines Öls mit einem Reingehalt von 97,6 % erhalten, was einer Ausbeute von 96,6 % der Theorie entspricht.

Beispiel 8

In ein Gemisch aus 202,5 g (1 Mol) 2,4-Dinitrochlorbenzol und 250 ml Ethylglykol werden innerhalb von 30 Minuten 193,5 g (1,4 Mol) wasserfreies Kaliumcarbonat portionsweise eingetragen. Die Temperatur steigt während dieser Zeit von 20 °C auf ca. 45 °C. Anschließend wird 3 Stunden bei 80 °C gerührt.

Danach wird das Reaktionsgemisch mit 1400 ml Wasser verdünnt. Das Zielprodukt scheidet sich als untere Phase ölig ab. Nach Abtrennen der wäßrigen Phase werden 253,7 g 2,4-Dinitro-1,-[2-ethoxy-ethoxy]-benzol mit einem Reingehalt von 98,3 % erhalten, was einer Ausbeute von 97,4 % entspricht.

$Kp_4$: 182-184 °C

Beispiel 9

Ein Gemisch aus 300 ml n-Butylglykol, 202,5 g (1 Mol) 2,4-Dinitrochlorbenzol und 207,3 g (1,5 Mol) Kaliumcarbonat wird unter Rühren innerhalb von 30 Minuten auf 100 °C erwärmt und 4 Stunden bei dieser Temperatur gehalten. Die Aufarbeitung des Ansatzes erfolgt analog Beispiel 8. Es werden 268,8 g 2,4-Dinitro-1- [2-butoxy -ethoxy]-benzol mit einem Reingehalt von 98,8 % erhalten, was einer Ausbeute von 93,5 % entspricht.

$Kp_4$: 193-194 °C

Beispiel 10

In ein Gemisch aus 101,3 g (0,5 Mol) 2,4-Dinitrochlorbenzol und 250 ml Methylglykol werden 244,4 g (0,75 Mol) wasserfreies Cäsiumcarbonat in Anteilen derart eingetragen, daß die Temperatur während dieser Zeit von 25 °C auf 60 °C steigt. Bereits nach 40-minütigem Rühren bei 60 °C ist vollständiger Umsatz eingetreten.

Aus dem Ansatz werden anschließend im Vakuum ca. 120 ml Methylglykol abdestilliert, welche im Folgeansatz wieder eingesetzt werden können. Nach Verdünnen des Reaktionsgemisches mit ca. 400 ml Wasser fällt das Zielprodukt aus, während die anorganischen Salze in Lösung gehen.

Das auf einer Nutsche isolierte und mit Wasser neutral gewaschene Produkt gibt nach dem Trocknen 115,6 g 2,4-Dinitromethoxyethoxy-benzol (Reingehalt: 98,9 %) vom Schmelzpunkt 37 °C, was einer Ausbeute von 94,5 % entspricht.

Beispiel 11

In ein Gemisch aus 30,4 g (0,15 Mol) 2,4-Dinitrochlorbenzol und 150 ml Methylglykol werden portionsweise 52,0 g (0,225 Mol) wasserfreies Rubidiumcarbonat eingetragen. Durch Außenkühlung wird verhindert, daß eine Temperatur von 60 °C überschritten wird. Eine nach 1-stündigen Rühren bei 60 °C aus dem Reaktionsgemisch entnommene Probe zeigt, daß bereits vollständiger Umsatz eingetreten ist.

Das anschließend in ca. 500 ml Wasser gefällte und auf einer Nutsche isolierte Produkt ergibt nach dem Trocknen 35,3 g 2,4-Dinitro-methoxyethoxy-benzol (Reingehalt: 98,7 %) vom Schmelzpunkt 36 - 37 °C, was einer Ausbeute von 96,0 % der Theorie entspricht.

**Patentansprüche**

**1.** Verfahren zur Herstellung von 2,4-Dinitrophenylethern der allgemeinen Formel (1)

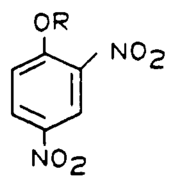

(1),

in welcher R eine Alkyl($C_1$-$C_6$)- oder Alkoxy($C_1$-$C_4$)-alkyl($C_1$-$C_4$)-Gruppe bedeutet, dadurch gekennzeichnet, daß man 1 Mol 2,4-Dinitrochlorbenzol in dem für die Etherbildung benötigten wasserfreien Alkohol der allgemeinen Formel (2)

R - OH (2),

in welcher R die vorstehend genannte Bedeutung hat, in Gegenwart von 1,0 bis 3,0 Mol eines wasserfreien Alkalimetallcarbonats, bei Temperaturen von 20 °C bis 150 °C, ggf. unter Druck, umsetzt.

**2.** Verfahren nach Anspruch 1, dadurch gekennzeichnet, daß man mit 1,05 bis 1,8 Mol eines wasserfreien Alkalimetallcarbonats umsetzt.

**3.** Verfahren nach mindestens einem der Ansprüche 1 und 2, dadurch gekennzeichnet, daß man in Gegenwart von wasserfreiem Kaliumcarbonat umsetzt.

**4.** Verfahren nach mindestens einem der Ansprüche 1-3, dadurch gekennzeichnet, daß man bei Temperaturen von 40 °C bis 120 °C umsetzt.

**5.** Verfahren nach mindestens einem der Ansprüche 1-4, dadurch gekennzeichnet, daß man als wasserfreien Alkohol der genannten Formel (2) wasserfreies Methanol, Ethanol, Propanol, Butanol, Methylglykol oder Ethylglykol einsetzt.

**6.** Verfahren nach mindestens einem der Ansprüche 1-3, dadurch gekennzeichnet, daß man in Gegenwart

eines inerten Verdünnungsmittels arbeitet.

**Claims**

**1.** A process for the preparation of 2,4-dinitrophenyl ethers of the formula (1)

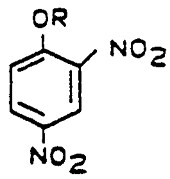

(1)

in which R denotes an alkyl($C_1$-$C_6$) or alkoxy($C_1$-$C_4$)-alkyl($C_1$-$C_4$) group, which comprises reacting 1 mole of 2,4-dinitrochlorobenzene in the anhydrous alcohol which is required for the ether formation and is of the formula (2)

R - OH (2)

in which R has the abovementioned meaning, in the presence of 1.0 to 3.0 mole of an anhydrous alkali metal carbonate, at temperatures of 20 ° C to 150 ° C, where appropriate under pressure.

**2.** The process as claimed in claim 1, wherein 1.05 to 1.8 mole of an anhydrous alkali metal carbonate is used for the reaction.

**3.** The process as claimed in at least one of claims 1 and 2, wherein the reaction is carried out in the presence of anhydrous potassium carbonate.

**4.** The process as claimed in at least one of claims 1-3, wherein the reaction is carried out at temperatures of 40 ° C to 120 ° C.

**5.** The process as claimed in at least one of claims 1-4, wherein anhydrous methanol, ethanol, propanol, butanol, methylglycol or ethylglycol is used as anhydrous alcohol of the said formula (2).

**6.** The process as claimed in at least one of claims 1-3, which is carried out in the presence of an inert diluent.

**Revendications**

**1.** Procédé pour préparer des éthers 2,4-dinitrophényliques répondant à la formule générale 1 :

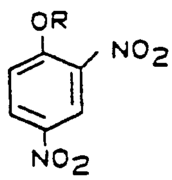

(1)

dans laquelle R représente un alkyle en $C_1$-$C_6$ ou un alcoxy($C_1$-$C_4$)-alkyle($C_1$-$C_4$), procédé caractérisé en ce qu'on fait réagir 1 mol de 2,4-dinitro-chlorobenzène dans l'alcool anhydre nécessaire pour la formation de l'éther, cet alcool répondant à la formule générale 2 :

R - OH (2)

dans laquelle R a la signification qui lui a été donnée ci-dessus, en présence de 1,0 à 3,0 mol d'un carbonate de métal alcalin anhydre, à des températures de 20 à 150 ° C, éventuellement sous pression.

**2.** Procédé selon la revendication 1 caractérisé en ce qu'on fait réagir avec de 1,05 à 1,8 mol d'un carbonate de métal alcalin anhydre.

**3.** Procédé selon l'une des revendications 1 et 2, caractérisé en ce qu'on fait réagir en présence de carbonate de potassium anhydre.

**4.** Procédé selon l'une quelconque des revendications 1 à 3, caractérisé en ce qu'on effectue la réaction à des températures de 40 à 120 ° C.

**5.** Procédé selon l'une quelconque des revendications 1 à 4, caractérisé en ce qu'on utilise, comme alcool anhydre répondant à la formule 2 mentionnée, du méthanol, de l'éthanol, du propanol, du butanol, du méthylglycol ou de l'éthylglycol, anhydres.

**6.** Procédé selon l'une quelconque des revendications 1 à 3, caractérisé en ce qu'on opère en présence d'un diluant inerte.